# EUROPEAN PATENT SPECIFICATION

(11) **EP 2 935 021 B1**
(45) Date of publication and mention of the grant of the patent: **19.04.2017**
(21) Application number: 13811186.9
(22) Date of filing: 17.12.2013
(51) Int. Cl.: B65B 55/08, A61L 2/08

(54) **DEVICE AND METHOD FOR STERILIZING PACKAGING CONTAINERS BY ELECTRON BEAM**
VORRICHTUNG UND VERFAHREN ZUR STERILISIERUNG VON VERPACKUNGSBEHÄLTERN MIT ELEKTRONENSTRAHLEN
DISPOSITIF ET PROCÉDÉ POUR STÉRILISER DES RÉCIPIENTS D'EMBALLAGE PAR FAISCEAU D'ÉLECTRONS

(30) Priority: 20.12.2012 EP 12198586; 17.01.2013 SE 1350054; 01.02.2013 SE 1350127; 04.03.2013 SE 1350256; 25.06.2013 SE 1350773
(43) Date of publication of application: 28.10.2015
(73) Proprietor: Tetra Laval Holdings & Finance S.A., 1009 Pully (CH)
(72) Inventor: HANSEN, Fredrik, S-237 36 Bjärred (SE); NÄSLUND, Lars-Åke, S-244 65 Furulund (SE); DICKNER, Jonas, S-253 51 Påarp (SE); EIDEBAKKEN, Roy, S-247 60 Veberöd (SE); ZETTERSTRÖM, Håkan, S-241 31 Eslöv (SE); ÅKESSON, Mats, S-213 63 Malmö (SE)
(74) Representative: Tetra Pak - Patent Attorneys SE
(86) International application number: PCT/EP2013/076874
(87) International publication number: WO 2014/095842

(56) References cited:
- WO-A1-2011/011079
- US-A1- 2012 219 455

## Description

### FIELD OF THE INVENTION

The present invention relates to an irradiation device for sterilizing packaging containers with electron beams. The invention also relates to a method.

### BACKGROUND OF THE INVENTION

Within the food industry, it is common practice to pack liquid and partly liquid food products in packaging containers manufactured from a packaging laminate comprising a core layer of paper or paperboard and one or more barrier layers of, for example, polymer material or aluminium foil.

An increasingly common packaging type is the "carton bottle" manufactured in a filling machine in that packaging blanks of the above-described packaging laminate are formed and sealed as a sleeve. Said sleeve is closed in one end in that a top of thermoplastic material is injection moulded directly on the sleeve end portion. The sheets of packaging laminate may be cut from a magazine reel of packaging laminate.

When the top is finished the packaging container is ready to be filled with product through the still open bottom, and then sealed and finally folded. Before the filling operation the packaging container undergoes treatment. If distribution and storage is to be made in chilled temperature the packaging container is disinfected, whereas if distribution and storage is to be made in ambient temperature, the packaging container needs to be sterilized, i.e. become aseptic. A conventional way of sterilizing a ready-to-fill packaging container is to use hydrogen peroxide, preferably in gas phase.

Another way to sterilize such packaging containers is to irradiate it by means of a low voltage electron beam emitted from an electron beam emitter. An example of linear irradiation by electron beam of ready-to-fill packaging containers is disclosed in the international patent publication WO 2005/002973. The electron beam emitter is cylindrical with an electron exit window positioned at one of the distal ends. The packaging container is lifted to surround the electron beam emitter during the sterilization cycle. Other examples of irradiation of packaging containers, in these cases PET bottles, are described in for example WO 2011/011079 and EP 2 371 397. In these systems emitters are used having a diameter small enough to be passed through a neck portion of the bottles.

### SUMMARY OF THE INVENTION

The present invention relates to a sterilization device for sterilizing packaging containers with electron according to claim 1.

In one or more embodiments the packaging container conveying means is adapted to displace the packaging container in relation to the first electron beam emitter between a first position in which the packaging container and the first electron beam emitter are not engaged with each other and a second position in which the packaging container and the first electron beam emitter are fully engaged with each other.

In one or more embodiments the carrier comprises a packaging container in-feed position and a packaging container out-feed position, and wherein said packaging container conveying means, upon conveying a packaging container from the packaging container in-feed position to the packaging container out-feed position, is at the same time adapted to displace the packaging container in relation to the first electron beam emitter from the first position to the second position and back to the first position, for thereby sterilizing the interior of the packaging container.

In one or more embodiments more than one first electron beam emitter are equally distributed to the periphery of the carrier.

In one or more embodiments it comprises two second electron beam emitters, arranged opposite each other in such a way that the packaging containers can pass in between them, and having their electron exit windows facing the packaging containers. The two second electron beam emitters have their longitudinal centre axes parallel to each other, and one of the two second electron beam emitters is positioned at or in the vicinity of a centre shaft of the carrier, and the other second electron beam emitter is arranged in the vicinity of the carrier periphery.

In one or more embodiments it comprises two second electron beam emitters, arranged opposite each other in such a way that the packaging containers can pass in between them, wherein the two second electron beam emitters have their longitudinal centre axes parallel to each other, and wherein one of the two second electron beam emitters is positioned with its longitudinal centre axis aligned with a centre shaft of the carrier, and the other second electron beam emitter is arranged in the vicinity of the carrier periphery.

In one or more embodiments the two second electron beam emitters are arranged such in relation to the carrier that the exterior sterilization is performed before the packaging containers arrive at the out-feed position.

In one or more embodiments the interior sterilization of the packaging container finishes when the packaging container passes the at least one second electron beam emitter such that an electron cloud of the first electron beam emitter at least partly overlaps an electron cloud of the at least one second electron beam emitter, said electron clouds together forming a combined electron cloud.

In one or more embodiments the opening of the packaging container is at least temporarily positioned within the combined cloud.

In one or more embodiments said first electron beam emitter is being adapted to at least sterilize any mutual contact surfaces between the packaging container and said packaging container conveying means before said packaging container conveying means is brought into contact with the packaging container or when the packaging conveying means release the packaging container.

In one or more embodiments one or more second electron beam emitter is arranged in the vicinity of the carrier, with the electron exit window facing towards the centre of the carrier, and wherein the packaging container conveying means is adapted to rotate each packaging container around its own axis at least when passing said one or more second electron beam emitter.

In one or more embodiments at least two second electron beam emitters are arranged, with their electron exit windows opposite each other, in such a way that the packaging containers can pass in between them, and in such a way that the exterior sterilization is performed when the packaging containers are no longer in any engagement with the first electron beam emitter.

The invention also relates to a method for sterilizing packaging containers with electron beams according to claim 11.

In one or more embodiments the method comprises the step of finalizing the interior sterilization such that an electron cloud emitted from the first electron beam emitter partly overlaps the electron cloud emitted from the at least one second electron beam emitter, said electron clouds together forming a combined electron cloud.

In one or more embodiments the method comprises the step of at least temporarily positioning the opening of the packaging container within the combined cloud.

### BRIEF DESCRIPTION OF THE DRAWINGS

In the following, an embodiment of the invention will be described in greater detail, with reference to the enclosed very schematic drawings, in which:
Fig. 1 a is a view of a packaging container and an exemplary first electron beam emitter, for sterilizing the interior of the packaging container, in a fully engaged sterilization position,
Fig. 1b is a view of an alternative packaging container and first electron beam emitter,
Fig. 1c is a view of the first electron beam emitter and its electron cloud,
Fig. 2a is a perspective view of a second electron beam emitter for sterilizing the exterior of the packaging container,
Fig. 2b is a perspective view of a cathode which may be used in the electron beam emitter of Fig. 2a,
Fig. 2c is a cross section of the cathode of Fig. 2b,
Fig. 2d is a view of two second electron beam emitters being arranged opposite each other, and their electron cloud.

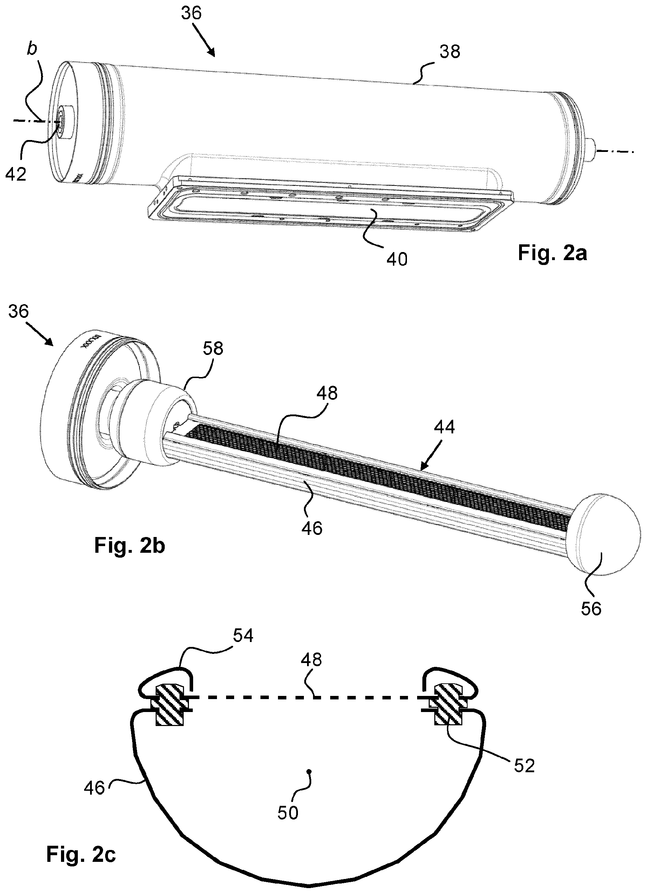

Fig. 2c is a view of the volume of an interface region between two second electron beam emitters.

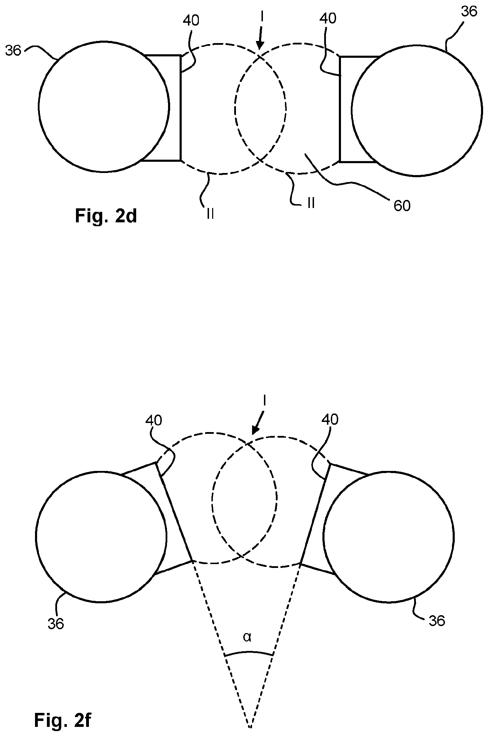

Fig. 2f is a view of two second electron beam emitters having electron exit windows being inclined relative each other.

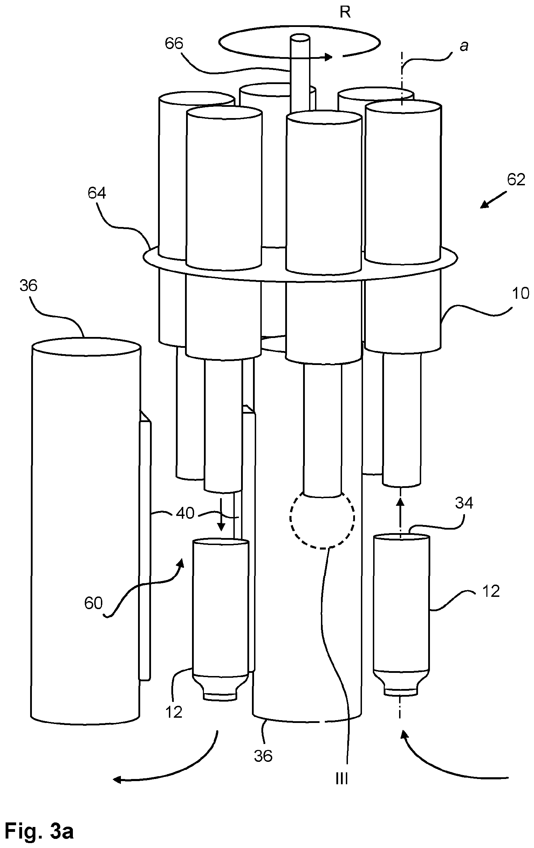

Fig.3a is a perspective view of a first embodiment of an irradiation device not of the invention together with two packaging containers, and

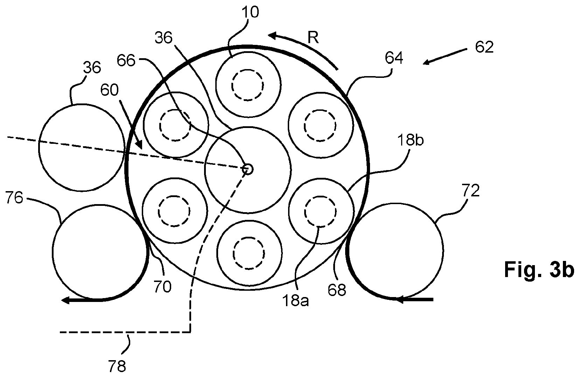

Fig. 3b is a top view of the irradiation device of Fig. 3a.

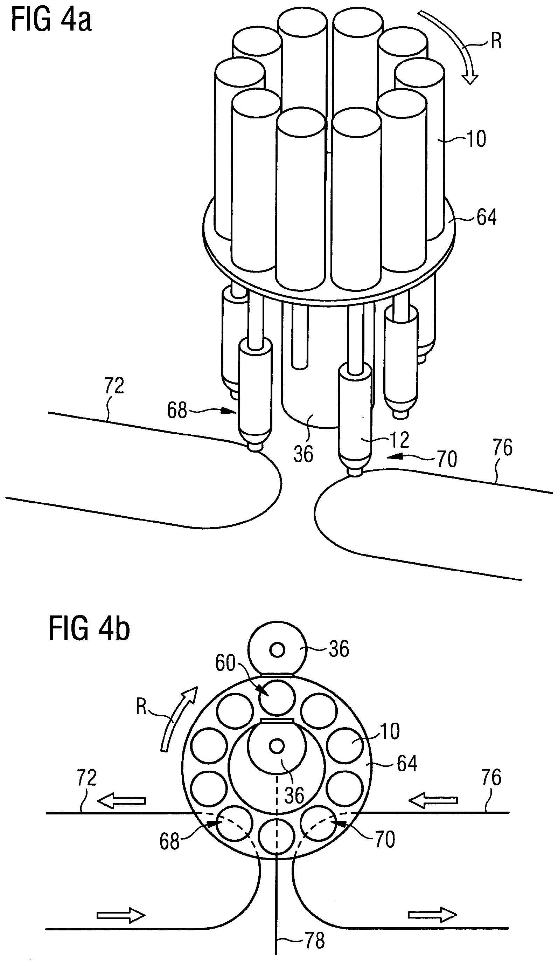

Fig. 4a is a perspective view of a second embodiment of an irradiation device not of the invention together with some packaging containers.
Fig. 4b is a top view of the irradiation device of Fig. 4a.

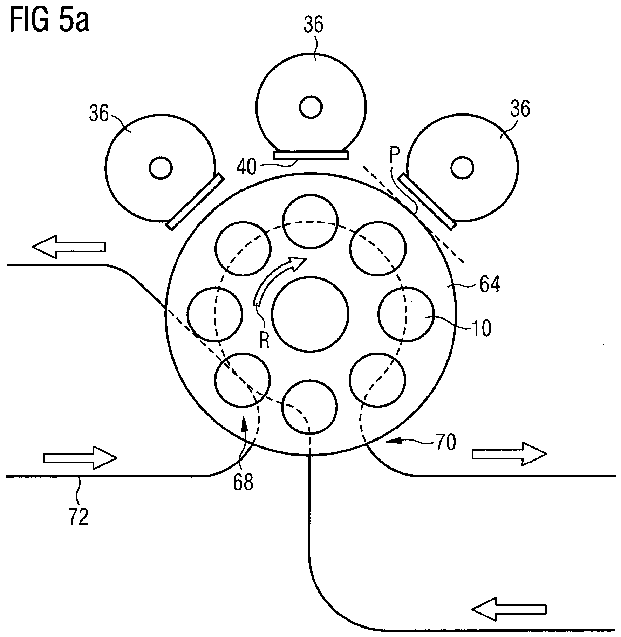

Fig. 5a is a top view of a third embodiment of an irradiation device not of the invention.

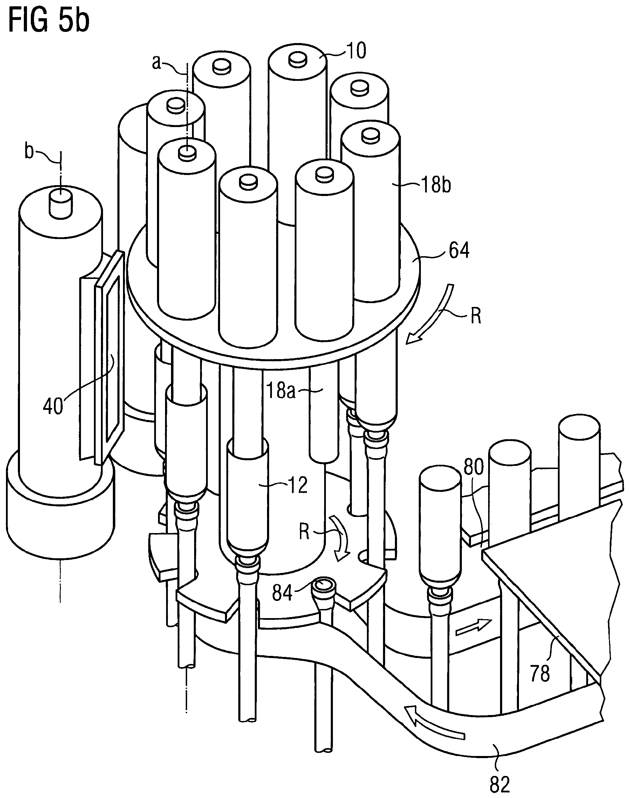

Fig. 5b is a perspective view of the irradiation device in Fig. 5a together with some packaging containers.

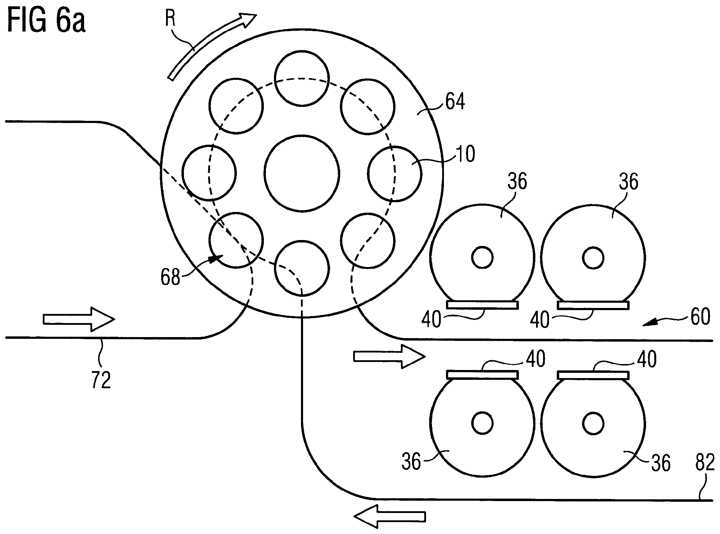

Fig. 6a is a top view of a fourth embodiment of an irradiation device not of the invention together with some packaging containers.

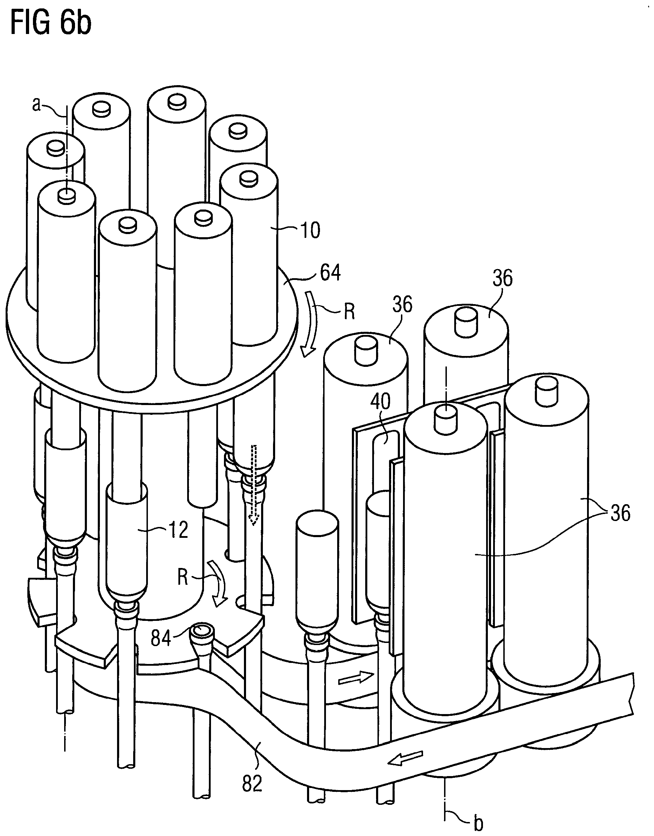

Fig. 6b is a perspective view of the irradiation device in Fig. 6a.

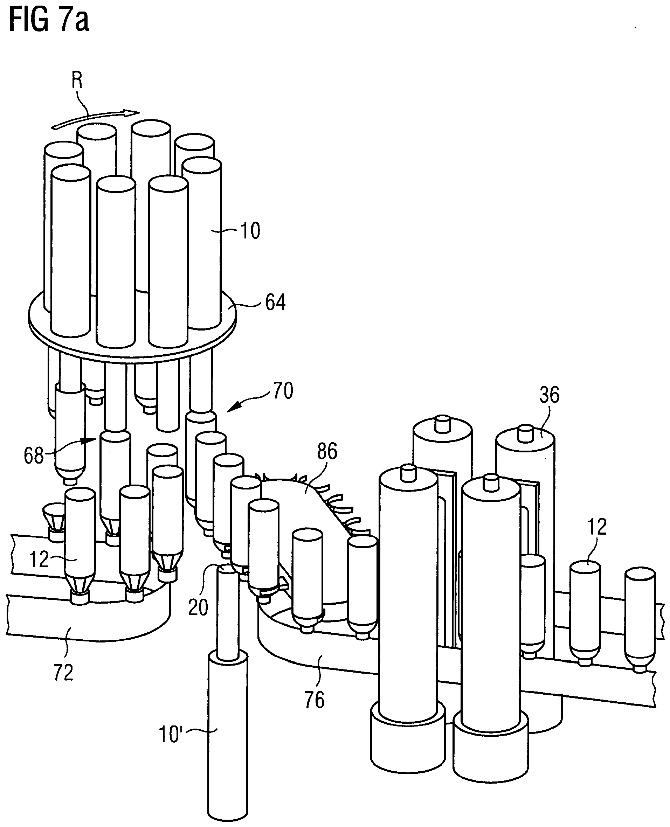

Fig. 7a is a top view of a fifth embodiment of an irradiation device not of the invention together with some packaging containers.

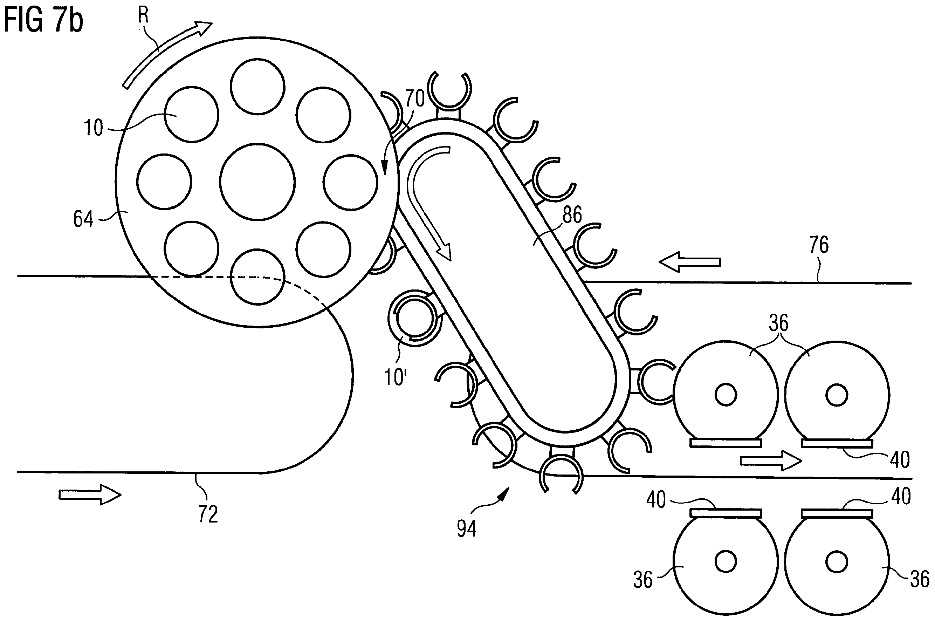

Fig. 7b is a perspective view of the irradiation device in Fig. 7a.

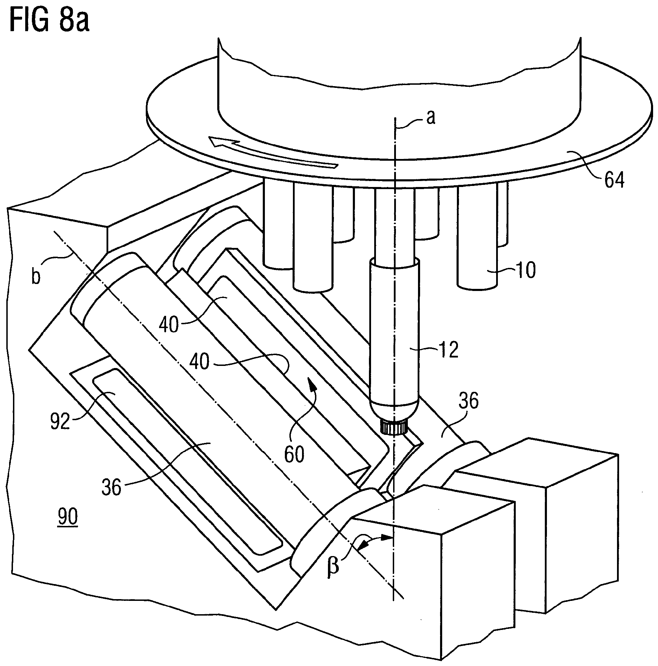

Fig. 8a is a perspective view of a sixth embodiment of the irradiation device of the invention together with one packaging container.

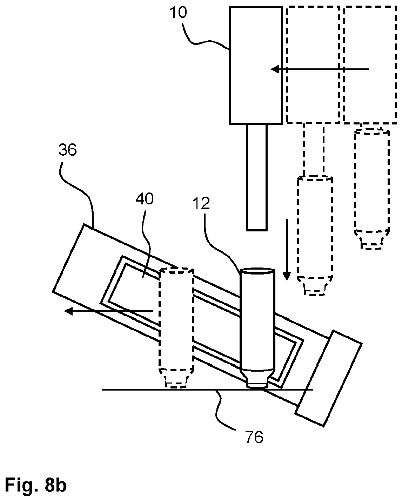

Fig. 8b is an illustration of the packaging container movement through the interface region of the sixth embodiment.

### DETAILED DESCRIPTION OF A PREFERRED EMBODIMENT

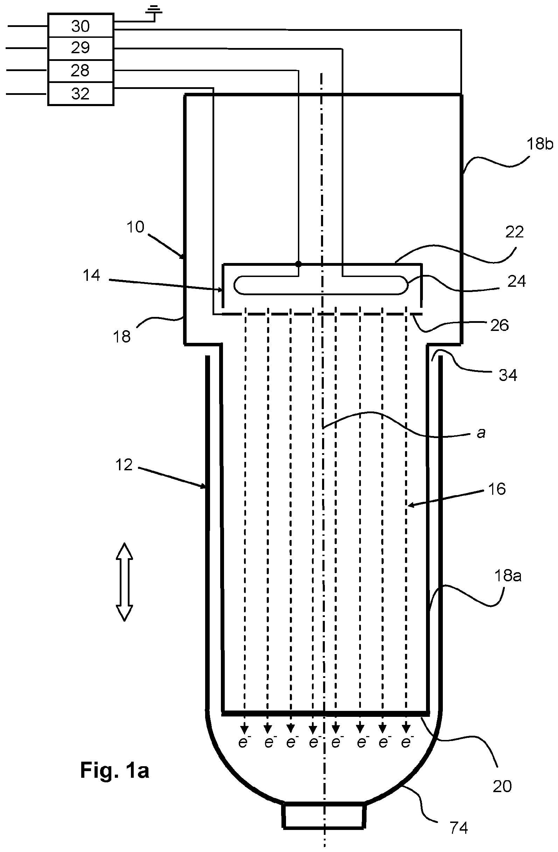

In the following, and with reference to Figure 1a, an exemplary first electron beam emitter 10 for sterilizing the interior of ready-to-fill packaging containers 12 and the concept of electron beam sterilization will be briefly described.

The electron beam emitter 10 comprises an electron generator 14 for emitting a substantially circular electron beam 16 along a path. The electron generator 14 is enclosed in a hermetically sealed vacuum chamber 18. Said vacuum chamber 18 is provided with an electron exit window 20.

The electron generator 14 comprises a cathode housing 22 and a filament 24. Optionally, the electron generator 14 also comprises a control grid 26. In use, an electron beam 16 is generated by heating the filament 24. When an electrical current is fed through the filament 24, the electrical resistance of the filament 24 causes the filament to be heated to a temperature in the order of 2000°C. This heating causes the filament 24 to emit a cloud of electrons. The electrons are accelerated towards the electron exit window 20 by means of a high-voltage potential between the cathode housing 22 and the exit window 20 (being the anode). Further, the electrons pass through the electron exit window 20 and continue towards the target area, i.e. in this case the inside of the packaging container 12.

The high-voltage potential is created by for example connecting the cathode housing 22 and the filament 24 to a power supply 28 and by connecting the vacuum chamber to ground 30. The filament also needs a second connection 29. The first electron beam emitter 10 is generally denoted low voltage electron beam emitter if the voltage is below 300 kV. In the disclosed design the accelerating voltage is in the order of 95 kV. This voltage results in a kinetic (motive) energy of 95 keV in respect of each electron. However, another voltage can be chosen, for example in the interval 75-150 kV. By applying an electrical potential also to the control grid 26 the emission of electrons may be further controlled. If a separate and variable electrical potential is applied to the control grid 26 it makes it possible to use the control grid 26 for active shaping of the generated electron beam. For these purposes the control grid 26 may be electrically connected to a separate power supply 32.

The filament 24 can be made of tungsten. The grid 26, placed between the filament 24 and an electron beam exit window 20 provided with a number of openings and is used for diffusing the electron beam 16 into a more uniform beam, and for focusing the electron beam 16 towards the target area.

The emitter 10 is, as mentioned, further provided with an electron exit window 20. The window 20 can be made of a metallic foil, such as for example titanium, and can have a thickness in the order of 4-12 µm. A supporting net (not shown) formed of aluminum or copper supports the foil from inside the vacuum chamber 18. The electrons are exiting the vacuum chamber 18 through the exit window 20.

In this embodiment the vacuum chamber 18 is made up of two cylindrical bodies 18a, 18b with substantially circular cross sections. An end of the first cylindrical body 18a is provided with the electron exit window 20. The diameter of said first body 18a is small enough to be inserted into the ready-to-fill packaging container 12, the cross section of said first body is dimensioned such that it can be guided through an opening 34 of the packaging container 12. The second body 18b is provided with the electron beam generator14, and the diameter of said second body 18b is larger than the first body 18a. The diameter of the emitted electron beam 16, while still inside the emitter 10, is smaller than the diameter of the first body 18a.

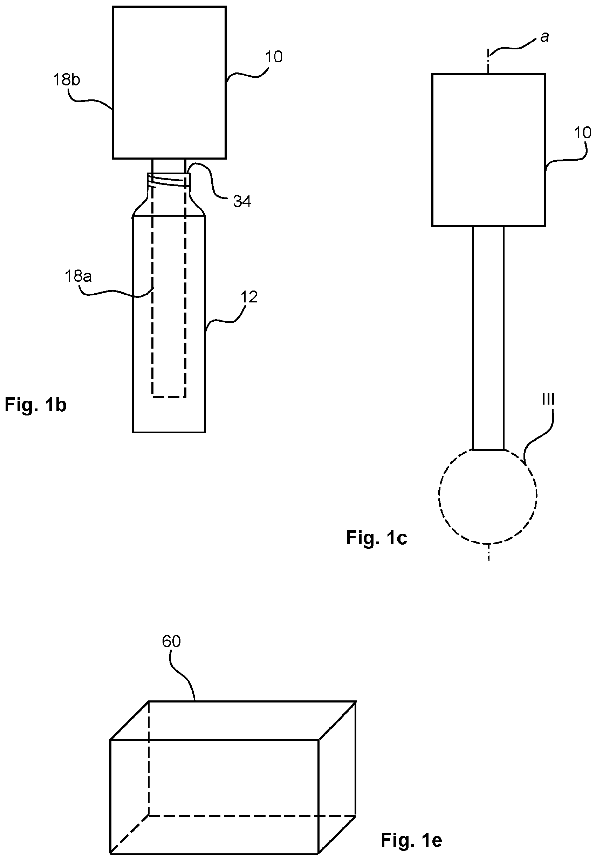

In Figure 1a the opening 34 of the packaging container is an open bottom end, which after filling will be sealed and folded to form a substantially flat bottom surface. It should however be understood that the opening, through which the first electron beam device is received, may in other embodiments be arranged in the top of the packaging container, constituting a neck or spout portion of the packaging container. Figure 1b illustrates such. The neck or spout portion will, after filling, be sealed by for instance a screw cap.

The first electron beam emitter 10 is adapted to generate an electron cloud III. A cross section of the electron cloud III is represented by a dashed line III in Figure 1c. The cross sectional shape is circular, as shown, or droplet-shaped. The electron cloud is axis-symmetrical, around axis *a*, and the cloud volume is thereby spherical (or droplet-shaped).

Typically, for a first electron beam emitter 10 of the above described type, the dose rate in air at the boundary of this electron cloud III is approx. 1000-1600 kGy/s. In the centre of the electron cloud the dose rate is higher. The energy of the first electron beam emitter 10 needs to be matched with the sterilization time available and the packaging container size and shape. Within the dashed line III the electron beam should at least have enough energy to kill any microbiological material travelling through it at a decided maximum velocity.

Fig. 2a is a perspective view of an exemplary hermetically sealed second electron beam emitter 36 for sterilizing the exterior of the packaging containers 12. The purpose of the drawing is simply to illustrate the basic components of the emitter, and it should be emphasized that the purpose is not to provide a true constructional drawing or in any other way limit the present invention.

The main component of the electron beam emitter is the tubular body 38, which has an elongate shape. An electron exit window 40 provides an outlet for electrons from the vacuum inside the tubular body 38. The exit window 40 in turn comprises subassemblies not relevant for the present invention, yet having the properties of providing an outlet window for electrons while preserving vacuum inside the body 38. A proximal end of the body 38 comprises an assembly including electrical connections 42.

Fig. 2b shows the second electron beam emitter 36 without the tubular body 38, and a cathode 44 is shown. The cathode 44 comprises a cathode housing 46, which is also shown in the very schematic cross section of Fig. 2c. The cathode housing 46 is formed as a semi-annular shell, the open side of which is covered by a control grid 48. Inside the annular shell of the cathode housing 46 one or more filaments 50 (see Fig. 2c) are arranged, extending from a proximal end of the cathode housing 46 to a distal end thereof. In use, an electron beam is generated by heating the filament 50, using a current, and by accelerating the electron towards the electron exit window 40 by means of a high-voltage potential between the cathode housing 46 and the exit window 40 (being the anode). The high-voltage potential is created by for example connecting the cathode housing to a power supply and by connecting the tubular body to ground. By applying an electrical potential also to the control grid 48 the emission of electrons may be further controlled. This can be achieved by connecting the control grid 48 to a separate power supply.

The second electron beam emitter 36 has an accelerating voltage in the order of 95 kV. This voltage results in a kinetic (motive) energy of 95 keV in respect of each electron. However, another voltage can be chosen, for example in the interval 75-150 kV.

The control grid 48 comprises a flat perforated surface comprising a pattern of openings or through-holes for passage of electrons. The open side of the cathode housing 46, carrying the control grid 48, should for obvious reasons be facing the electron exit window 40. The cathode housing 46 and the control grid 48 are mounted together by means of attachment means 52. If there is a difference in electrical potential between the cathode housing 46 and the grid 48 said attachment means 52 are preferably electrical isolator elements. Free longitudinal end portions 54 of the control grid 48 are bent in a direction towards each other, i.e. in a lateral direction being perpendicular to the extension of the longitudinal end portions, to form bulge-like shapes for the formation of electron beam shaping electrodes. Such electrodes are sometimes referred to as "Wehnelt" electrodes. The bulge-like shape will assist in the generation of a smooth predictable electrical field to the benefit of performance of the electron beam emitter. They help shaping the electric field so that the electrons will hit the exit window 40 in an essentially right angle, i.e. in a direction essentially perpendicular to the plane of the exit window 40.

The described cathode is fitted into the electron beam emitter as shown in Fig. 2b. The proximal end as well as the distal end of the cathode housing 46 comprises electrical connections as well as physical suspensions for the filament 50. At the distal end this arrangement is housed inside or covered with a dome-shaped cap 56. At its proximal end the cathode housing 46 is suspended to the elongate body and the suspension is encapsulated by an annular cover 58.

In Fig. 2d is shown two second electron beam emitters 36, arranged opposite each other. The electron beam generated by each of the second electron beam emitters 36 during operation forms a combined electron cloud I. The boundary of the cross section of that electron cloud is shown with dashed line I in figure. The electron cloud of each of the second electron beam emitters 36 is denoted II. Each cloud II has a substantially circular cross section. The combined electron cloud I at least fills an interface region 60 between the electron exit windows 40 of the two electron beam emitters 36. Said interface region 60 is illustrated by a three dimensional volume shown in Fig. 2c. The electron cloud I forms a volume being large enough to at least cover the volume of said interface region 60, the cloud I at least fills the volume of Fig. 2c. The longer sides are approximately as large as the longitudinal extension of the electron exit window 40 of the second electron beam emitter 36. Within the boundaries of the electron cloud I the electron beams have at least enough energy to kill any microbiological material travelling through the interface region at the same average velocity as the velocity of the packaging containers or of any particle or air flow travelling through the same. Typically, for a second electron beam emitter 36 of the above described type, the dose rate in air at the boundary of the electron cloud II is approx. 400-800 kGy/s. In the centre of the electron cloud I, II the dose rate is higher. The size of the interface region 60 and the energy of the second electron beam emitters 36 will need to be matched for each application such that the electron cloud I at least covers the interface region 60 and such that the minimum dose rate of the electron cloud I is enough to kill any microbiological particles passing the interface region 60 at a chosen maximum velocity.

In the arrangement shown in Fig. 2d the second electron beam emitters 36 are arranged opposite each other with their electron beam exit windows 40 facing each other and the interface region 60. The planes of the electron exit windows 40 are parallel to each other. In an alternative arrangement the planes of the electron exit windows 40 are slightly inclined in relation to each other. An example of such arrangement is shown in Fig. 2f. An angle α is provided between the two electron exit windows 40.

In Fig. 3a and 3b a first embodiment of an irradiation device 62 not of the invention is shown. It is provided with several first electron beam emitters 10 of the type used for interior sterilization of packaging containers 12. Such first electron beam emitters 10 were described above with reference to Fig. 1 a.

In this first embodiment six first electron beam emitters 10 are provided to a rotatable carrier 64. In other embodiments the number of first emitters 10 may be different, for example the number can be in the range from 4 to 20 depending on the space provided and the speed of the filling machine.

The rotatable carrier 64 is, in this embodiment, shaped as a wheel and is rotatable round a centre shaft 66. The direction of the rotation is illustrated by the arrow R and the rotatable movement is continuous. The first emitters 10 are equally distributed to the periphery of the carrier 64, and are fixed to the carrier 64 so that they are being carried along when the carrier 64 rotates.

The irradiation device 62 further comprises first packaging container conveying means, not shown, being adapted to convey the packaging container 10, from a carrier in-feed position 68 to a carrier out-feed position 70, synchronously with the carrier revolution movement and in alignment with the first electron beam emitter 10. In this embodiment this means that the packaging container 12 is moved synchronously with the first electron beam emitter 10 and that a longitudinal centre axis of the packaging container 12 is aligned with a longitudinal centre axis of the electron beam emitter 10, see the dashed-dotted line *a* in Fig. 3a. From that figure, as well as from Fig. 1a, it is also seen that the opening 34 in the packaging container 12 is concentric with the centre axes of the packaging container 12 and first electron beam emitter 10.

The first packaging container conveying means is being further adapted to vertically displace the packaging container 12 in relation to the first electron beam emitter 10. In the embodiment shown the first electron beam emitters 10 are arranged stationary in the carrier 64 and cannot move towards the packaging container 12. Due to the heavy weight, the fragile electron exit window 20 and the high voltage connections, or electrical cables, it is an advantage to have the first emitters 10 stationary in the vertical direction, and instead move the packaging containers.

The first packaging container conveying means displace the packaging container 12 between a first position in which the packaging container 12 and the first electron beam emitter 10 are not engaged with each other and a second position in which the packaging container 12 and the first electron beam emitter 10 are fully engaged with each other. When the packaging container 12 and the first electron beam emitter 10 are engaged the packaging container 12 has been raised to a position in which it surrounds the first electron beam emitter 10 and the first electron beam emitter 10 is sterilizing the interior of the packaging container 12. When they are not engaged the packaging container is positioned underneath the first electron beam emitter 10, i.e. the packaging container 12 has not started to surround the emitter 10, or has just been displaced down from the fully engaged position. At the in-feed and out-feed positions 68, 70 the packaging container 12 is positioned in the first position, i.e. not in engagement with the first electron beam emitter 10.

At the in-feed position 68 the packaging containers 12 are supplied to the irradiation device 62 from a conveyor 72. Each packaging container 12 is aligned with a corresponding first electron beam emitter 10 and is gripped by means of gripping means comprised in the first packaging container conveying means.

When the carrier 64 rotates, so that the first electron beam emitter 10 and the packaging container 12 rotates from the in-feed position 68 to the out-feed position 70, the first packaging container conveying means displaces the packaging container 12 towards the first electron beam emitter 10 for performing inside surface sterilisation.

The initial step of the sterilisation, i.e. the initial part of the packaging container sterilisation cycle, will now be described in more detail. Before the first packaging container conveying means is brought into contact with the packaging container 12 at or in the vicinity of the conveyor 72, the first electron beam emitter 10 is used for at least sterilizing any mutual contact surfaces between the packaging container 12 and the first packaging container conveying means. Mutual contact surfaces are surfaces of the packaging container 12 and the conveying means that will be in contact with each other once the gripping means of the first packaging container conveying means has gripped the packaging container 12. The electron cloud III of the first electron beam emitter 10 is used for sterilizing at least the surfaces of the first packaging container conveying means which will come into contact with the packaging container 12, i.e. at least portions of the gripping means. Similarly, the first electron beam emitter 10 will also be used for sterilizing at least the surfaces on the packaging container 12 that will become in contact with the gripping means of the first packaging container conveying means. The contact surfaces of the packaging container 12 may be either interior or exterior areas or a combination, depending on the type of packaging container conveying means and how it is supposed to grip the packaging container 12. The contact surfaces are near the opening 34, i.e. the contact surfaces are in the end of the packaging container facing the first electron beam emitter 10. Of course also surfaces nearby will be sterilized, or at least irradiated to some extent.

As mentioned above the sterilization of the mutual contact surfaces is made before the first packaging container conveying means is being brought into contact with the packaging container 12. This is any moment before the actual contact takes place. When a first electron beam emitter 10 and a packaging container 12 are present in the in-feed position 68 the first packaging container conveying means is displaced towards the packaging container 12. During this displacement the mutual contact surfaces are sterilized. Although the design of the electron beam 16 from the first emitter 10 is adapted for sterilizing the interior of the packaging container 12, the electron cloud III generated near the electron exit window 20 can be utilised for sterilization of the mutual contact surfaces, and other nearby surfaces, of the first packaging container conveying means and the packaging container 12. The cloud III is large enough to cover the contact surfaces.

When the gripping means of the first packaging container conveying means has gripped the packaging container 12 the packaging container 12 is started being displaced towards the first electron beam emitter 10. Since the first electron beam emitter 10 is aligned with the opening 34 of the packaging container 12 the first electron beam emitter 10 is received in the packaging container 12. Hence, sterilization of the interior of the packaging container 10 is made.

Somewhere between the in-feed and out-feed positions 68, 70 the packaging container 12 has been displaced such that the packaging container 12 is fully engaged with the first electron beam emitter 10. The fully engaged second position is shown in Fig. 1a. In that position the innermost area of the packaging container 12 is sterilized, in this case a top portion 74 of the packaging container 12.

The sterilization cycle of the inside surface is completed when the packaging container 12 reaches the out-feed position 70, although the actual sterilization may have been completed before reaching the out-feed position 70.

When the packaging container 12 reaches said out-feed position 70, the packaging container 12 is retracted, i.e. lowered, or has already been lowered from the second position back to the first position. As mentioned above the packaging container 12 is then ready to be fed out from the irradiation device 62 by means of a conveyor 76.

It has been previously described that the mutual contact surfaces between the packaging container and the first conveying means is made before the first conveying means grips the packaging container. In an alternative embodiment the mutual surfaces of the packaging container and the first conveying means can be sterilized upon releasing the packaging container, i.e. when the first conveying means releases its grip of the packaging container. In addition, such mutual surface sterilization can obviously be made both before the packaging container comes into contact with the first conveying means and after the conveying means has released the packaging container.

Upon further rotation of the carrier 64, from the out-feed position 70 and back to the in-feed position 68, the electron beam emitter 10 is not engaged with any packaging container 12 but is still maintained in operation, i.e. it is still emitting the same electron beam 16. The space between the out-feed and in-feed positions may be utilized for a sensor device, for measuring one or several dose parameters of the first electron beam emitters 10. By such the functionality of the first electron beam emitters are measured between every sterilization cycle. When reaching the in-feed position 68 again a new sterilization cycle is commenced with a new packaging container 12 supplied from the conveyor 72 and the in-feed position 68.

The conveyors 72, 76 at the in-feed and out-feed positions as such are not the focus of this invention and will therefore not be described in detail. At the in-feed position 68 the packaging container 12 is transferred from the conveyor 72 to the carrier 64. In this embodiment the conveyor 72 is a star wheel. At the out-feed position 70 the packaging container 12 is, as mentioned above, transferred from the carrier 64 to the conveyor 76 for further transport to a filling station. Like the conveyor 72, the conveyor 76 is a star wheel in this embodiment.

The first packaging container conveying means as such is not the focus of this invention either and will therefore not be described in detail. It may be arranged on the carrier 64, or on the first electron beam emitters 10, or a combination thereof. It may alternatively be arranged separate from the carrier 64 but able to convey the packaging containers 12 synchronous with the carrier rotation. For example it may be arranged on an irradiation shielding device enclosing the carrier 64. The first packaging container conveying means is, as mentioned above, provided with packaging container gripping means that is adapted to grip the packaging container 12. The gripping means are preferably positioned underneath, and close to, the electron exit window 20 when not gripping a packaging container 12, so that it is continuously sterilized by the electron cloud III present near the electron exit window 20. For achieving the relative vertical movement towards and away from the electron beam emitters 10 the first packaging container conveying means may comprise a cam curve, servomotor or similar that guides the gripping means in the vertical direction upon rotation of the carrier 64. To be able to obtain the benefit of mutual contact surface sterilization before gripping, the gripping means are preferably adapted to grip the packaging container 12 at or near the opening 34.

In order for a packaging container to for example reach a sterilization level referred to as "commercially sterile" an absorbed dose of approximately 25kGy is needed in every point of the interior surface of the packaging container. In the embodiment the interior sterilization is performed during a relative movement between the packaging container and the first electron beam emitter. Hence, the dose has to be calculated by the dose rate (dose delivery per time unit, mentioned as kGy/s) delivered by the emitter and the time each portion of the interior surface of the packaging container is exposed to the electron cloud. An increase in speed of the relative movement will lead to a decrease of the time available for the interior sterilization, and in order to maintain the same dose the dose rate of the emitter needs to be increased. Similar, if the speed of the relative movement is decreased, the time available for sterilization will increase and the dose rate of the emitter will have to be decreased in order not to overexpose the interior surface. In this context any air flows or turbulences near the packaging container need to be taken into account. Air flowing faster than the speed of the relative movement will affect the sterilization result. Such airflow may make a microorganism travel too fast past the electron cloud, which may lead to that microorganism is not being killed. Hence, it is important to control the velocity of the airflows around the packaging container such that it is not higher than the speed of the relative movement. An important aspect is also the flow created in the packaging container because of the relative movement. When the packaging container is lowered from the first electron beam emitter air will automatically be sucked into the packaging container, creating backflow, i.e. an airflow into the packaging container which in some cases may be disturbing for the sterilization effect. To obtain an efficient sterilization and a secure process it has been found that a preferred movement profile comprises a first stage of quickly raising the packaging container to surround the first electron beam emitter, and then a second stage of slowly lowering the packaging container. In other words, the packaging container is moved with a first velocity from the first position to the second position, and with a second velocity from the second position back to the first position, said second velocity being lower than the first velocity. Although the first electron beam emitter is continuously in operation, i.e. continuously emitting electrons, the interior sterilization is considered to be made during the slow lowering of the packaging container. The slow lowering should be slow enough to not create air flows into the packaging container having a velocity higher than that of the relative movement. The volume of the gap between the packaging container and the first electron beam emitter as well as the volume of the first electron beam emitter are factors that influence the velocity of the flows created into the packaging container.

In the embodiment described the first stage of quickly raising the packaging container to surround the first electron beam emitter will take approximately half the time compared to the time spent for the second stage of slowly lowering the packaging container. Hence, the first velocity is approximately twice as high as the second velocity.

Only the interior sterilisation has been described so far. However, the sterilization cycle described above also comprises sterilization of the outside surface of the packaging container 12. In this embodiment said outside sterilization is performed by means of two second electron beam emitters 36 of the type described above in relation to Figures 2a-2c. The second electron beam emitters 36 are arranged in a stationary position between the packaging container in-feed position 68 and the packaging container out-feed position 70, in such a way that it can irradiate the exterior of each passing packaging container 12. As can be seen in the figures one of said second emitters 36 is arranged in the centre of the irradiation device 62 with its longitudinal axis aligned, or parallel, with the carrier centre shaft 66. The other second emitter 36 is arranged with its longitudinal axis parallel to the centre emitter 36, but is arranged in the periphery of the carrier 64. The second electron beam emitters 36 have their flat, elongated electron exit windows 40 positioned opposite each other, with a passage in between for the inside surface electron beam emitters 10 and the thereto engaged packaging containers 12. This passage is referred to as the previously described interface region 60.

The width of the electron beam from the second electron beam emitter 36, along the longitudinal axis of the emitter 36, is larger than the longitudinal extension, i.e. height, of the packaging containers 12. The electron beams from the second emitters 36 are directed transverse to the tangent of the packaging container transportation direction, and are directed in mutually opposite directions. As can be seen from the figures the second emitters 36 are, in this embodiment, arranged in the end of the sterilization cycle, near the packaging container out-feed position 70.

The second electron beam emitters 36 are adapted to sterilize the exterior surface of the packaging container 12 before they enter into an aseptic zone. The interface region 60 between the two electron beam emitters 36 defines the entrance to the aseptic zone. A packaging container 12 being present in the aseptic zone needs to be fully sterilised or needs to have an electron cloud acting as a barrier between any still unsterile portion of the packaging container and the aseptic zone.

In this first embodiment the second electron beam emitters 36 sterilize the exterior of the packaging container 12 just before the first electron beam emitter 10 finish the interior sterilization of the packaging container 12. The inside sterilisation is finished off by lowering the packaging container 12 back to the first position and the electron cloud III from the first emitter 10 is at least partly overlapping the electron cloud I formed by the second electron beam emitters 36 to form a combined cloud I, III. The opening 34 of the packaging container is at least temporarily positioned within the combined cloud.

After the passage between the two second electron beam emitters 36 it is time for the first packaging container conveying means to deliver the packaging container 12 to a subsequent conveying means. In the embodiment shown the conveyor 76 is such subsequent conveying means. The conveyor 76 is adapted to convey the packaging containers 12 directly, or by means of further subsequent conveyors, to a filling station for filling content into the packaging containers 12. The filling station is comprised in the aseptic zone. The first packaging container conveying means of the irradiation device 62 will release the packaging containers and bring them into contact with the conveyor 76. Upon releasing the packaging container 12 the electron cloud III of the first electron beam emitter 10 is again sterilising the contact surfaces. As a consequence both a portion of the inside surface and the outside surface of the packaging container 12 is sterilised, and it is secured that the packaging container 12 is fully sterilised.

In Fig. 3b the conveyor 76 is shown as a wheel, but it may be of any kind, for example it may alternatively be an endless packaging container transport conveyor.

As previously mentioned the area from the interface region 60 (between the second electron beam emitters 36) and the out-feed position 70 of the irradiation device is part of the aseptic zone. To separate the zone before the interface region, being a non-aseptic zone, from the aseptic zone the irradiation device 62 comprises an aseptic barrier 78. The aseptic barrier 78 is illustrated by a dashed line in Fig. 3b. The aseptic barrier 78 can be a physical barrier, for example a wall, a gas flow barrier or preferably a combination of the two. The function of the aseptic barrier 78 is to prevent particles and microbiological material to reach the aseptic zone.

Further, one or more air flow barriers and/or one or more physical barriers are provided to protect the aseptic zone from parts of the first packaging container conveying means that are not sterilized and will be moving through or above the aseptic zone.

The irradiation device 62 has been described in a schematic way. Only parts of the irradiation device 62 being involved in the invention has been described, but it is to be understood that the irradiation device 62 comprises also additional parts such as drive units for driving the carrier 64 and the first packaging container conveying means, and irradiation shielding enclosing the irradiation device 62 for securing that electrons and x-rays are not spread to the environment outside of the device 62.

With regard to this first embodiment (Figs. 3a and 3b) it has been described that the packaging container 12 is movable relative the first electron beam emitter 10 by means of the first packaging container conveying means. Alternatively, the first electron beam emitters 10 may be made movable in the vertical direction such that they may be displaced towards the packaging container 12. Another alternative is that both of the packaging container 12 and the first electron beam emitter 10 are displaced a distance each. Further, the carrier 64 has been described as being rotatable with a continuous movement. Alternatively, the carrier 64 may instead rotate intermittently. Further, the carrier 64 is a wheel in the shown embodiment. It may of course be an endless carrier of have any shape. Furthermore, the second emitters 36 are arranged in the end of the sterilization cycle, near the packaging container out-feed position 70. In another embodiment they may be arranged in the beginning of the sterilization cycle, closer to the in-feed position 68.

The movement that the packaging container 12 undergoes in relation to the first electron beam emitter 10 during the sterilization cycle, moving from the first position to the second position and then back again to the first position, may be made with different timings from what have been described in relation to the above embodiment. For example, the movement towards the first electron beam emitter 10 maybe started later than described, i.e. not at the in-feed position, but when the packaging container has been conveyed some towards the out-feed position. Similarly, the packaging container may return to the first position simultaneously as reaching the out-feed position, or it's alternatively retracted some or all the way from the second position to the first position at the out-feed position. Furthermore, said movement may be made continuous or made in a stepwise way.

It has been described that two second, oppositely positioned, electron beam emitters 36 are sterilizing the outside surface of the packaging containers 12. Alternatively, for example, it is possible to use only one second electron beam emitter 36. In such case, to obtain full sterilization of the entire outside surface, it is preferred to rotate the packaging container 12 around its own axis. In that case a wall or similar may act as a boundary of the interface region 60, such that the interface region 60 extends from the single second electron beam emitter 36 to said wall (instead of to an opposing second electron beam emitter).

It has been described that the packaging container 12 and the first electron beam emitter 10 make a relative movement in relation to each other to perform inside surface sterilization. Although the first electron beam emitter 10 is operated continuously the actual sterilization may be performed either when the packaging container is moved upwards to surround the first electron beam emitter, i.e. when the packaging container is displaced towards the fully engaged position, or it is performed when the packaging container is moved downwards from the fully engaged position. The speed of the packaging container movement is adjusted accordingly. Alternatively, the inside surface sterilization is made partly in both direction, i.e. part of the dose is delivered during the upward movement and part of the dose is delivered during the downward movement of the packaging container.

It should be pointed out that the electron cloud III forms an irradiation lock or aseptic lock inside the packaging container 12 during sterilization. Although most of the interior sterilization is made outside the aseptic chamber, the electron cloud III is able to form an aseptic lock inside the packaging container 12. No dirt or microbiological material or particle can manage to travel through the cloud III and into the sterilized interior of the packaging container 12. The volume of the electron cloud III of the first emitter 10 covers the opening 34 of the packaging container. Hence, the sterility of the interior, below the electron cloud III, can be assured even during the time when the packaging container 12 is still outside the aseptic zone.

Figs. 4a-4b show a second embodiment of an irradiation device not of the invention. The second embodiment is to a large extent similar to the first embodiment, and only the differences will be described. The same reference numerals as in the previously described embodiment will be used for similar features.

In this embodiment the carrier 64 comprises ten first electron beam emitters 10. At an in-feed position 68 the packaging containers 12 are delivered from a conveyor 72 to first packaging container conveying means (not shown) arranged in connection with the carrier 64. At an out-feed position 70 the first packaging container conveying means releases the packaging container 12 and the packaging container is received in a conveyor 76, which will transport the packaging containers to the filling station. In this second embodiment the conveyors 72 and 76 are endless conveyor belts with gripping means for gripping the packaging containers 12 for example around their sleeves. Their movement direction is illustrated by arrows in the figures, and it should be noted that the conveyors 72, 76 are only partly shown in the figures.

The first packaging conveying means grips the packaging container 12 on the packaging container outside surface close to the opening 34.

Two second electron beam emitters 36 are positioned half way between the in-feed position 68 and the out-feed position 70. The rotation direction of the carrier 64 is clockwise in figures 4a-4b and represented by the arrow R. At the in-feed position 68 the packaging containers 12 are started to be lifted towards the first electron beam emitters 10, and when the packaging container 12 is passing the interface region 60 between the two second electron beam emitters 36 the packaging container 12 is in the fully engaged position with regard to the first electron beam emitter 10. After having passed the interface region 60, and being in the aseptic zone, the packaging container 12 is lowered from the first electron beam emitter 10. Before reaching the out-feed position 70 the inside sterilization is finished. When the first packaging container conveying means releases the packaging container 12 the first electron beam emitter 10 is sterilizing the upper outside surface (near opening 34) at which the first packaging conveying means was in contact with the packaging container 12. The area between the in-feed and out-feed positions 68, 70 is provided with an aseptic barrier 78 such as for example a wall to separate the non-aseptic zone on the left hand side of Fig. 4b from the aseptic zone on the right hand side. Part of the aseptic barrier 78 may be an air flow barrier, for instance the part of the barrier 78 shown with a dashed line in Figure 4b. In Fig. 4a the aseptic barrier has been omitted.

Figs. 5a-5b show a third embodiment of an irradiation device not of the invention. The third embodiment is to a large extent similar to the second embodiment, and only the differences will be described. The same reference numerals as in the previously described embodiments will be used for similar features.

In the third embodiment the first packaging container conveying means comprises means for rotating each packaging container 12 around its own longitudinal axis. Instead of having two opposing second electron beam emitters 36 with an interface region 60 in between, this embodiment comprises three second electron beam emitters 36 arranged at the periphery of the carrier 64 with their electron exit windows 40 directed in a direction towards the center of the carrier 64. The plane of the electron exit window 40 is parallel to a tangent of the carrier periphery, said tangent originating from a point *p* centrally in front of the electron exit window, see Fig. 5a. Longitudinal axes, exemplified by dashed-dotted line b in Fig. 5b, of the second electron beam emitters 36 are parallel to the longitudinal axes, exemplified by dashed-dotted line *a* in Fig. 5b, of the first electron beam emitters 10. At least when passing said three second electron beam emitters 36 the packaging container 12 is adapted to rotate around its own axis, in order to become sterilized around its entire outside surface.

In this embodiment the first packaging conveying means is adapted to grip the packaging container 12 in the cap. The gripping is made by grippers 84. This facilitates the rotation of the packaging container 12 around its own axis.

The inside surface sterilization is finished after finishing the outside surface sterilization. When the first electron beam emitter 10 is finishing the inside surface sterilization it will automatically as well sterilize a portion of the outside surface near the opening 34, to make surface that the entire packaging container 12 except for the cap is sterilized.

The first packaging container conveying means does not hand over the packaging containers 12 to another conveyor at the out-feed position 70 but continues to convey the packaging container 12 further, for example all the way to the filling station. The first packaging container conveying means is shown as a belt 82 in the figures. The previously described grippers 84 cooperate with said belt 82.

Since neither the caps nor the first packaging conveying means are sterilized, the aseptic zone needs to have a lower boundary or barrier. This means that the cap portion of the packaging containers 12, the first packaging container conveying means and the air volume surrounding them cannot be part of the aseptic zone, but need to be separated from it. For this a barrier in the form of a laminar flow of sterile air is provided. The flow is directed from top to bottom of the aseptic zone, i.e. downwards in Fig. 5b, in the direction from the packaging container opening 34 to the cap. This secures that the aseptic zone stays sterile and that the packaging container 12 stays sterile (except for the cap).

The flow barrier may be strengthened by a physical barrier. Such is provided in the form of a wall 78, part of which is shown in the figures. Said wall 78 is formed as a horizontal plane which separates the upper aseptic zone from the lower non-aseptic zone. The wall 78 has a slot 80 for allowing the packaging containers 12 to be conveyed by the first packaging container conveying means. The top-down directed laminar flow acts as an aseptic barrier in the slot 80 preventing dirt and microbiological material from entering the aseptic zone from below, i.e. from the non-aseptic zone situated underneath the aseptic zone.

Additional barriers, physical and flow barriers, are preferably provided between the in-feed conveyor 72 and the belt 82, although these are omitted in the figures.

It should as well be noted that Fig. 5a also shows a line 72 illustrating a conveyor feeding packaging containers to the in-feed position 68 of the irradiation device. The movement of said conveyor is shown by arrows. Similarly, the movement of the belt 82 is illustrated by arrows, and the movement of the carrier 64 is illustrated by the arrow R. The belt 82 and the conveyor 72 are endless, but are only partly shown.

The grippers 84 are adapted to perform the raising and lowering of the packaging containers needed for performing the inside surface sterilization, however any drive means for the displacement is not shown. Such drive means may comprise a cam curve, servo motors or similar.

A fourth embodiment not of the invention is shown in Figs. 6a-6b. The fourth embodiment is to a large extent similar to the third embodiment, and only the differences will be described. The same reference numerals as in the previously described embodiments will be used for similar features.

This fourth embodiment differs from the third embodiment in that the second electron beam emitters 36 are arranged after, i.e. downstream of, the carrier 64 and that the first packaging container conveying means is not rotating each packaging container 12 around its own axis.

Two pairs of second electron beam emitters 36 are arranged opposite each other with a passage in between for packaging containers 12. The passage is to be considered as the previously described interface region 60. The electron exit windows 40 of the second electron beam emitters 36 are facing the passage. The planes of the electron exit windows 40 are all parallel to each other. Longitudinal axes, exemplified by dashed-dotted line b, of the second electron beam emitters 36 are parallel to the longitudinal axes, exemplified by dashed-dotted line *a*, of the first electron beam emitters 10.

The sterilization of the packaging containers 12 is made in two steps, in a first step the first packaging container conveying means is adapted to transport the packaging containers 12 synchronously with the carrier 64 to let the first electron beam emitters 10 sterilize the inside surface of the packaging containers 12. As in the previous embodiments the packaging container 12 is lifted to surround the first electron beam emitter 10 for the inner sterilization, and then lowered again before or upon leaving the carrier 64. An arrow in dashed line illustrates the lowering motion.

In a second step the first packaging container conveying means is adapted to transport the packaging containers 12 past the second electron beam emitters 36 for sterilization of the outside surface of the packaging containers 12. The second sterilization step is performed right after the first sterilization step.

In the figures it is shown that the first packaging conveying means is adapted to grip the packaging container 12 in the cap, like in the third embodiment. The first packaging container conveying means is shown as a belt 82 with grippers 84, like in the third embodiment. However, because of the different arrangement of the second electron beam emitters 36, it is not needed that the packaging containers 12 in this fourth embodiment are rotated around their own axes. The second electron beam emitters 36 are able to sterilize the outside surface of the packaging container 12 despite any rotation.

However, similar to the third embodiment, the cap is not entirely sterilized by the second electron beam emitters 36 because of the cap's contact with the gripper 84 of the first packaging container conveying means during the outside surface sterilization. An aseptic barrier in the form of a laminar flow and possibly also a physical barrier in the form of a wall, examples of which are described in relation to the third embodiment, may be provided in the area downstream of the second electron beam emitters 36 where the aseptic zone starts.

It should as well be noted that Fig. 6a also shows a line 72 illustrating a conveyor feeding packaging containers to the in-feed position 68 of the irradiation device. The movement of said conveyor is shown by arrows. Similarly, the movement of the belt 82 is illustrated by arrows, and the movement of the carrier 64 is illustrated by the arrow R. The belt 82 and the conveyor 72 are endless, but are only partly shown. The grippers 84 are adapted to perform the raising and lowering of the packaging containers needed for performing the inside surface sterilization, however any drive means for the displacement is not shown. Such drive means may comprise a cam curve, servo motors or similar.

In Figs. 7a-7b a fifth embodiment not of the invention is shown. The fifth embodiment is to a large extent similar to the fourth embodiment, and only the differences will be described. The same reference numerals as in the previously described embodiments will be used for similar features.

The differences between the fourth and fifth embodiments are that the fifth embodiment has a different packaging container conveying and an additional first electron beam emitter 10'. At an in-feed position 68 first packaging container conveying means (not shown) are gripping the packaging containers 12 from a an in-feed conveyor 72. The first packaging container conveying means is gripping the cap of the packaging container 12 as described in relation to for example the fourth embodiment. The first packaging container conveying means conveys the packaging containers 12 during the inside surface sterilization taken place in the carrier 64. At the out-feed position 70 the packaging containers 12 are fetched by an intermediate conveyor 86. The intermediate conveyor 86 grips the packaging container 12 such that the cap is exposed, for example it grips around the sleeve. With the help of the intermediate conveyor 86 the packaging container 12 is conveyed past the additional first electron beam emitter 10'. The additional first electron beam emitter 10' is arranged such that its electron exit window 20 is facing the caps of the packaging containers 12, i.e. it is arranged underneath the intermediate conveyor 86 with its electron exit window 20 facing upwards. The electron exit window surface 20 is parallel to the axial surface of the cap. By means of said additional first electron beam emitter 10' the caps are sterilized.

The intermediate conveyor 86 hands over the packaging containers 12 to a second conveying means 76 that grips the packaging containers 12 in the cap. The handover is taking place at a handover point 94 just after the cap sterilization, and the second conveying means 76 is arranged partly underneath the intermediate conveyor 86. The second conveying means 76 is sterilized and is moving in and out of the aseptic zone.

The second conveying means 76 conveys the packaging containers 12 past the second electron beam emitters 36. The second electron beam emitters 36 are arranged as described in relation to the fourth embodiment.

The second conveying means 76 transports the packaging containers 12 to the filling station, or hands over the packaging containers 12 to a subsequent conveyor that transports the packaging containers 12 to filling.

The conveyors 72 and 76 are endless, but are only partly shown in the figures.

In Figs. 8a and 8b a sixth embodiment of the irradiation device of the invention is shown. The sixth embodiment is to a large extent similar to the previous embodiments, and the following description will only focus on the arrangement of the second electron beam emitters 36. The same reference numerals as in the previously described embodiments will be used for similar features.

In the sixth embodiment two second electron beam emitters 36 are arranged with their electron exit windows 40 facing each other. They are arranged either as shown in Fig. 2d or as shown in Fig. 2f. However, they are arranged with their longitudinal axes inclined an angle β in relation to the vertical direction, i.e. being the same as the longitudinal axes of the first electron beam emitters 10. The longitudinal axis of one second electron beam emitter is illustrated by the dashed-dotted line *b* and the longitudinal axis of one of the first electron beam emitters is illustrated by the dashed-dotted line *a* in Fig. 8a. Both lines are shown visible, for the sake of understanding, but is should of course be understood that both axes extend through the centre of their respective emitter.

In between the electron exit windows a passage, i.e. the interface region 60, is formed through which the packaging containers 12 are adapted to be passed each with its longitudinal axis parallel to the vertical direction.

Similar to the previous embodiments the packaging containers 12 are fed to the carrier 64 in at in-feed position (not shown). The carrier 64 rotates in the clockwise direction (see rotation direction dented R in Fig. 8a) and the packaging container 12 is raised to surround the first electron beam emitter 10 in order for the inside surface sterilisation to be performed. The embodiment further comprises first packaging container conveying means, not shown, being adapted to convey the packaging containers from the in-feed position to an out-feed position synchronously with the carrier revolution movement and in alignment with the first electron beam emitter 10. This is similar to what has been described in relation to the previous embodiments.

The packaging container 12 is raised to the fully engaged position, in relation to the first electron beam emitter 10, between the in-feed position and an out-feed position by means of the first packaging container conveying means. Said out-feed position is being the position where the packaging container 12 is leaving the carrier 64, and enters the interface region 60 between the second electron beam emitters 36.

Upon finalisation of the inside surface sterilization the packaging container 12 is retracted, i.e. lowered, from the engaged position on the first electron beam emitter 10 to a non-engaged position. In Fig. 8b the packaging container 12 shown with solid lines illustrates this position. The packaging containers illustrated with dashed lines show moments before and after. As can be seen the packaging container (illustrated by solid line) in this position is also partly in the interface region 60 and the screw cap of the packaging container 12 has already almost passed through the interface region 60.

When the packaging container 12 leaves the carrier 64 it is received in a second conveying means 76. In Fig. 8b the second conveying means 76 is only shown as a line. The second conveying means 76 conveys the packaging container 12 in a direction to the left in the figure. The movement is illustrated by the packaging container in dotted lines to the left. By this movement the packaging container 12 is passed through the interface region 60 and is fully sterilized on its outside surface including the screw cap.

In Fig. 8a there is shown parts of walls 90 separating the aseptic zone and from the irradiation zone surrounding the carrier 64. The interface region 60 between the second electron beam emitters 36 is the entrance to the aseptic zone. However, the second electron beam emitters 36 are arranged outside the walls 90, and the packaging containers 12 pass a slot 92 in the walls 90 right after having passed the interface region 60. It should be understood that additional walls 90 may be arranged as well as flow barriers to prevent re-contamination of sterilized surfaces.

The first packaging container conveying means as well as the second packaging container conveying means have not been described in detail nor shown, but it should be understood that they can be of any of the types described in relation to the previous embodiments. The first packaging container conveying means is, as previously described in relation to for example the first embodiment, provided with packaging container gripping means that is adapted to grip the packaging container. The first packaging conveying means may be arranged on the carrier, or on the first electron beam emitters, or a combination thereof. It may alternatively be arranged separate from the carrier but able to convey the packaging containers 12 synchronous with the carrier rotation. For example it may be arranged on an irradiation shielding device enclosing the carrier. The packaging container is for example gripped around the sleeve. For achieving the relative vertical movement towards and away from the electron beam emitters 10 the first packaging container conveying means may comprise a cam curve, servomotor or similar that guides the gripping means in the vertical direction upon rotation of the carrier 64. The second conveying means may for instance be a drive belt having grippers for gripping the packaging containers in the screw caps or tops.

Although the present invention has been described with respect to an embodiment, it is to be understood that various modifications and changes may be made without departing from the object and scope of the invention as defined in the appended claims.

In the embodiments the packaging containers are carton bottles. However, it is easily realized that the invention can be applied also for other types of bottles, for example PET-bottles, for which sterilization and filling is made through the spout/neck. Similarly, the present invention is of course also applicable to sterilization of plastic bottle pre-forms, e.g. PET pre-forms. Sterilization of pre-forms is made before they are blow-moulded into a finished PET bottle. Hence, the interpretation of the word "packaging container" should in this regard be broad and comprises different types of containers such as for example carton-based packaging containers, plastic bottles and plastic pre-forms.

In the described embodiments sterilization and an aseptic zone have been described, which are suited for aseptic packaging. It should of course be understood that the invention can be used also in applications in which the packaging containers are only disinfected or hygienically treated, i.e. where the aim is not to reach an aseptic level, i.e. not aiming for commercial sterility. In such an embodiment the dose applied by the electron beam emitters is normally lower than the dose used for aseptic packaging. The emitters are then not sterilizing the packaging containers, but merely irradiating them to a desired level. The aseptic zone is in this case a clean zone.

In the embodiments shown the first electron beam emitters are stationary in the vertical direction, i.e. the relative movement between the first electron beam emitter and the packaging container is performed by the packaging container only. However, in an alternative embodiment the electron beam emitter 10 is moved and the packaging container is stationary in the vertical direction. The electron beam emitter is hence lowered into the open end of the packaging container.

## Claims

1. Sterilization device for sterilizing packaging containers (12) with electron beams, said sterilization device comprising
at least one first electron beam emitter (10) adapted for sterilization of at least the interior of the packaging container (12), and
at least one second electron beam emitter (36) adapted for sterilization of at least the exterior of said packaging container (12), wherein
the interior sterilization of the packaging container (12) is performed in that the first electron beam emitter (10) and the packaging container (12) are adapted to perform a relative movement in relation to each other, such that at least a portion of the first electron beam emitter (10), comprising an electron exit window (20), is received in the packaging container (12) through an opening (34) in the packaging container (12), and
the exterior sterilization of the packaging container (12) is performed in that the packaging container is adapted to pass an electron exit window (40) of the at least one second electron beam emitter (36),
wherein said sterilization device comprises a rotatable carrier (64) to which the at least one first electron beam emitter (10) is being arranged such that it is movable together with the carrier (64), and wherein it comprises packaging container conveying means adapted to convey the packaging container (12) synchronously with the rotational movement of the carrier (64), keeping a longitudinal axis of the first electron beam emitter (10) aligned with a longitudinal axis of the packaging container (12),
wherein said sterilization device comprises two second electron beam emitters (36), arranged opposite each other in such a way that the packaging containers (12) can pass in between them, wherein the two second electron beam emitters (36) have their longitudinal centre axes parallel to each other, but wherein the two second electron beam emitters (36) are positioned at least partly underneath the carrier (64) and have their longitudinal centre axes inclined an angle in relation to the longitudinal axes of the first electron beam emitters (10) and a centre shaft of the carrier (64).

2. Sterilization device according to claim 1, wherein the packaging container conveying means is adapted to displace the packaging container (12) in relation to the first electron beam emitter (10) between a first position in which the packaging container (12) and the first electron beam emitter (10) are not engaged with each other and a second position in which the packaging container (12) and the first electron beam emitter (10) are fully engaged with each other.

3. Sterilization device according to claim 2, wherein the carrier (64) it comprises a packaging container in-feed position (68) and a packaging container out-feed position (70), and wherein said packaging container conveying means, upon conveying a packaging container (12) from the packaging container in-feed position (68) to the packaging container out-feed position (70), is at the same time adapted to displace the packaging container (12) in relation to the first electron beam emitter (10) from the first position to the second position and back to the first position, for thereby sterilizing the interior of the packaging container (12).

4. Sterilization device according to any of the preceding claims, wherein more than one first electron beam emitter (10) are equally distributed to the periphery of the carrier (64).

5. Sterilization device according to any of the preceding claims, wherein it comprises two second electron beam emitters (36), arranged opposite each other in such a way that the packaging containers (12) can pass in between them, and having their electron exit windows facing the packaging containers, wherein the two second electron beam emitters (36) have their longitudinal centre axes parallel to each other, and wherein one of the two second electron beam emitters is positioned at or in the vicinity of a centre shaft (66) of the carrier (64), and the other second electron beam emitter is arranged in the vicinity of the carrier (64) periphery.

6. Sterilization device according any of the preceding claims 3-5, wherein the two second electron beam emitters (36) are arranged such in relation to the carrier (64) that the exterior sterilization is performed before the packaging containers (12) arrive at the out-feed position (70).

7. Sterilization device according to any of the preceding claims, wherein the interior sterilization of the packaging container (12) finishes when the packaging container (12) passes the at least one second electron beam emitter (36) such that an electron cloud (III) of the first electron beam emitter (10) at least partly overlaps an electron cloud (I; II) of the at least one second electron beam emitter (36), said electron clouds (I, III) together forming a combined electron cloud, and wherein the opening (34) of the packaging container is at least temporarily positioned within the combined cloud (I, III).

8. Sterilization device according to any of the preceding claims, wherein said first electron beam emitter (10) is being adapted to at least sterilize any mutual contact surfaces between the packaging container (12) and said packaging container conveying means before said packaging container conveying means is brought into contact with the packaging container (12) or when the packaging conveying means release the packaging container.

9. Sterilization device according to any of the preceding claims, wherein one or more second electron beam emitter (36) is arranged in the vicinity of the carrier (64), with the electron exit window (40) facing towards the centre of the carrier (64), and wherein the packaging container conveying means is adapted to rotate each packaging container (12) around its own axis at least when passing said one or more second electron beam emitter (36).

10. Sterilization device according to any of the preceding claims, wherein at least two second electron beam emitters (36) are arranged, with their electron exit windows (40) opposite each other, in such a way that the packaging containers (12) can pass in between them, and in such a way that the exterior sterilization is performed when the packaging containers (12) are no longer in any engagement with the first electron beam emitter (10).

11. Method for sterilizing packaging containers with electron beams using a sterilization device comprising a rotatable carrier (64) to which the at least one first electron beam emitter (10) is being arranged such that it is movable together with the carrier (64), and wherein it comprises packaging container conveying means adapted to convey the packaging container (12) synchronously with the rotational movement of the carrier (64), keeping a longitudinal axis of the first electron beam emitter (10) aligned with a longitudinal axis of the packaging container (12), wherein said sterilization device comprises two second electron beam emitters (36), arranged opposite each other in such a way that the packaging containers (12) can pass in between them, wherein the two second electron beam emitters (36) have their longitudinal centre axes parallel to each other, but wherein the two second electron beam emitters (36) are positioned at least partly underneath the carrier (64) and have their longitudinal centre axes inclined an angle in relation to the longitudinal axes of the first electron beam emitters (10) and a centre shaft of the carrier (64), wherein the method comprises the steps of
sterilizing at least the interior of the packaging container with at least one first electron beam emitter and
sterilizing at the least the exterior of the said packaging container with at least one second electron beam emitter, and wherein
the interior sterilization of the packaging container is performed in that the first electron beam emitter and the packaging container are adapted to perform a relative movement in relation to each other, such that at least a portion of the first electron beam emitter, comprising an electron exit window, is received in the packaging container through an opening in the packaging container, and
the exterior sterilization of the packaging container is performed in that the packaging container is adapted to pass an electron exit window of the at least one second electron beam emitter.

12. Method according to claim 11, wherein the method comprises the step of finalizing the interior sterilization such that an electron cloud (III) emitted from the first electron beam emitter (10) partly overlaps the electron cloud (I; II) emitted from the at least one second electron beam emitter (36), said electron clouds (I, III) together forming a combined electron cloud.

13. Method according to claim 12, wherein it comprises the step of at least temporarily positioning the opening of the packaging container within the combined cloud.

## Patentansprüche

1. Sterilisierungsvorrichtung zum Sterilisieren von Verpackungsbehältern (12) mit Elektronenstrahlen, wobei die Sterilisierungsvorrichtung Folgendes umfasst:
mindestens eine erste Elektronenstrahlemissionsquelle (10), die zur Sterilisierung von mindestens dem Inneren des Verpackungsbehälters (12) eingerichtet ist, und
mindestens eine zweite Elektronenstrahlemissionsquelle (36), die zur Sterilisierung von mindestens dem Äußeren des Verpackungsbehälters (12) eingerichtet ist, wobei
die innere Sterilisierung des Verpackungsbehälters (12) dadurch durchgeführt wird, dass die erste Elektronenstrahlemissionsquelle (10) und der Verpackungsbehälter (12) eingerichtet sind, eine Relativbewegung in Bezug zueinander durchzuführen, so dass mindestens ein Abschnitt der ersten Elektronenstrahlemissionsquelle (10), die ein Elektronenaustrittsfenster (20) aufweist, durch eine Öffnung (34) im Verpackungsbehälter (12) in den Verpackungsbehälter (12) aufgenommen wird, und
die äußere Sterilisierung des Verpackungsbehälters (12) dadurch durchgeführt wird, dass der Verpackungsbehälter eingerichtet ist, ein Elektronenaustrittsfenster (40) der mindestens einen zweiten Elektronenstrahlemissionsquelle (36) zu passieren, wobei die Sterilisierungsvorrichtung einen drehbaren Träger (64) aufweist, an dem die mindestens eine erste Elektronenstrahlemissionsquelle (10) angeordnet ist, so dass sie zusammen mit dem Träger (64) beweglich ist, und wobei sie ein Verpackungsbehälterbeförderungsmittel aufweist, das eingerichtet ist, den Verpackungsbehälter (12) synchron mit der Drehbewegung des Trägers (64) zu befördern, wobei eine Längsachse der ersten Elektronenstrahlemissionsquelle (10) mit einer Längsachse des Verpackungsbehälters (12) ausgerichtet gehalten wird,
wobei die Sterilisierungsvorrichtung zwei zweite Elektronenstrahlemissionsquellen (36) aufweist, die in einer solchen Weise einander gegenüberliegend angeordnet sind, dass die Verpackungsbehälter (12) zwischen ihnen hindurch gehen können, wobei die Längsmittelachsen der beiden zweiten Elektronenstrahlemissionsquellen (36) parallel zueinander verlaufen, wobei jedoch die beiden zweiten Elektronenstrahlemissionsquellen (36) mindestens teilweise unter dem Träger (64) angeordnet sind und ihre Längsmittelachsen unter einem Winkel in Bezug zu den Längsachsen der ersten Elektronenstrahlemissionsquellen (10) und einem Mittelschaft des Trägers (64) geneigt sind.

2. Sterilisierungsvorrichtung nach Anspruch 1, wobei das Verpackungsbehälterbeförderungsmittel eingerichtet ist, den Verpackungsbehälter (12) in Bezug auf die erste Elektronenstrahlemissionsquelle (10) zwischen einer ersten Position, in der der Verpackungsbehälter (12) und die erste Elektronenstrahlemissionsquelle (10) nicht miteinander in Eingriff stehen, und einer zweiten Position zu verschieben, in der der Verpackungsbehälter (12) und die erste Elektronenstrahlemissionsquelle (10) vollständig miteinander in Eingriff stehen.

3. Sterilisierungsvorrichtung nach Anspruch 2, wobei der Träger (64) eine Verpackungsbehälter-Einführungsposition (68) und eine Verpackungsbehälter-Ausführungsposition (70) aufweist, und wobei das Verpackungsbehälterbeförderungsmittel beim Befördern eines Verpackungsbehälters (12) von der Verpackungsbehälter-Einführungsposition (68) zur Verpackungsbehälter-Ausführungsposition (70) gleichzeitig eingerichtet ist, den Verpackungsbehälter (12) in Bezug auf die erste Elektronenstrahlemissionsquelle (10) von der ersten Position zur zweiten Position und zurück zur ersten Position zu verschieben, um dadurch das Innere des Verpackungsbehälters (12) zu sterilisieren.

4. Sterilisierungsvorrichtung nach einem der vorhergehenden Ansprüche, wobei mehr als eine erste Elektronenstrahlemissionsquelle (10) gleichmäßig am Umfang des Trägers (64) verteilt ist.

5. Sterilisierungsvorrichtung nach einem der vorhergehenden Ansprüche, wobei sie zwei zweite Elektronenstrahlemissionsquellen (36) aufweist, die in einer solchen Weise einander gegenüberliegend angeordnet sind, dass die Verpackungsbehälter (12) zwischen ihnen hindurch gehen können, und deren Elektronenaustrittsfenster zu den Verpackungsbehältern weisen, wobei die Längsmittelachsen der beiden zweiten Elektronenstrahlemissionsquellen (36) parallel zueinander verlaufen, und wobei eine der beiden zweiten Elektronenstrahlemissionsquellen am oder in der Nähe eines Mittelschafts (66) des Trägers (64) angeordnet ist, und das andere zweite Elektronenstrahlemissionsquelle in der Nähe des Umfangs des Trägers (64) angeordnet ist.

6. Sterilisierungsvorrichtung nach einem der vorhergehenden Ansprüche 3 bis 5, wobei die beiden zweiten Elektronenstrahlemissionsquellen (36) in Bezug zum Träger (64) so angeordnet sind, dass die äußere Sterilisierung durchgeführt wird, bevor der Verpackungsbehälter (12) an der Ausführungsposition (70) ankommt.

7. Sterilisierungsvorrichtung nach einem der vorhergehenden Ansprüche, wobei die innere Sterilisierung des Verpackungsbehälters (12) endet, wenn der Verpackungsbehälter (12) die mindestens eine zweite Elektronenstrahlemissionsquelle (36) passiert, so dass eine Elektronenwolke (III) der ersten Elektronenstrahlemissionsquelle (10) sich mindestens teilweise mit einer Elektronenwolke (I; II) der mindestens einen zweiten Elektronenstrahlemissionsquelle (36) überlappt, wobei die Elektronenwolken (I, III) zusammen eine vereinigte Elektronenwolke bilden, und wobei die Öffnung (34) des Verpackungsbehälters mindestens vorübergehend in der vereinigten Wolke (I, III) angeordnet ist.

8. Sterilisierungsvorrichtung nach einem der vorhergehenden Ansprüche, wobei die erste Elektronenstrahlemissionsquelle (10) eingerichtet ist, mindestens alle gegenseitigen Kontaktflächen zwischen dem Verpackungsbehälter (12) und dem Verpackungsbehälterbeförderungsmittel zu sterilisieren, bevor das Verpackungsbehälterbeförderungsmittel mit dem Verpackungsbehälter (12) in Kontakt gebracht wird oder wenn das Verpackungsbeförderungsmittel den Verpackungsbehälter freigibt.

9. Sterilisierungsvorrichtung nach einem der vorhergehenden Ansprüche, wobei eine oder mehrere zweite Elektronenstrahlemissionsquellen (36) in der Nähe des Trägers (64) angeordnet sind, wobei das Elektronenaustrittsfenster (40) zur Mitte des Trägers (64) weist, und wobei das Verpackungsbehälterbeförderungsmittel eingerichtet ist, jeden Verpackungsbehälter (12) mindestens dann um seine eigene Achse zu drehen, wenn er die eine oder die mehreren zweiten Elektronenstrahlemissionsquellen (36) passiert.

10. Sterilisierungsvorrichtung nach einem der vorhergehenden Ansprüche, wobei mindestens zwei zweite Elektronenstrahlemissionsquellen (36) mit ihren einander gegenüberliegenden Elektronenaustrittsfenstern (40) in einer solchen Weise, dass die Verpackungsbehälter (12) zwischen ihnen hindurch gehen können, und in einer solchen Weise angeordnet sind, dass die äußere Sterilisierung durchgeführt wird, wenn sich die Verpackungsbehälter (12) nicht mehr in irgendeinem Eingriff mit der ersten Elektronenstrahlemissionsquelle (10) befinden.

11. Verfahren zum Sterilisieren von Verpackungsbehältern mit Elektronenstrahlen unter Verwendung einer Sterilisierungsvorrichtung, die einen drehbaren Träger (64) aufweist, an dem die mindestens eine erste Elektronenstrahlemissionsquelle (10) angeordnet ist, so dass sie zusammen mit dem Träger (64) beweglich ist, und wobei sie ein Verpackungsbehälterbeförderungsmittel aufweist, das eingerichtet ist, den Verpackungsbehälter (12) synchron mit der Drehbewegung des Trägers (64) zu befördern, wobei eine Längsachse der ersten Elektronenstrahlemissionsquelle (10) mit einer Längsachse des Verpackungsbehälters (12) ausgerichtet gehalten wird, wobei die Sterilisierungsvorrichtung zwei zweite Elektronenstrahlemissionsquellen (36) aufweist, die in einer solchen Weise einander gegenüberliegend angeordnet sind, dass die Verpackungsbehälter (12) zwischen ihnen hindurch gehen können, wobei die Längsmittelachsen der beiden zweiten Elektronenstrahlemissionsquellen (36) parallel zueinander verlaufen, wobei jedoch die beiden zweiten Elektronenstrahlemissionsquellen (36) mindestens teilweise unter dem Träger (64) angeordnet sind und ihre Längsmittelachsen unter einem Winkel in Bezug zu den Längsachsen der ersten Elektronenstrahlemissionsquellen (10) und einen Mittelschaft des Trägers (64) geneigt sind, wobei das Verfahren die folgenden Schritte aufweist:
Sterilisieren mindestens des Inneren des Verpackungsbehälters mit mindestens einer ersten Elektronenstrahlemissionsquelle und
Sterilisieren mindestens des Äußeren des Verpackungsbehälters mit mindestens einer zweiten Elektronenstrahlemissionsquelle, und wobei die innere Sterilisierung des Verpackungsbehälters dadurch durchgeführt wird, dass die erste Elektronenstrahlemissionsquelle und der Verpackungsbehälter eingerichtet sind, eine Relativbewegung in Bezug zueinander durchzuführen, so dass mindestens ein Abschnitt der ersten Elektronenstrahlemissionsquelle, die ein Elektronenaustrittsfenster aufweist, im Verpackungsbehälter durch eine Öffnung im Verpackungsbehälter aufgenommen wird, und die äußere Sterilisierung des Verpackungsbehälters dadurch durchgeführt wird, dass der Verpackungsbehälter eingerichtet ist, ein Elektronenaustrittsfenster der mindestens einen zweiten Elektronenstrahlemissionsquelle zu passieren.

12. Verfahren nach Anspruch 11, wobei das Verfahren den Schritt des Beendens der inneren Sterilisierung aufweist, so dass sich eine Elektronenwolke (III), die von der ersten Elektronenstrahlemissionsquelle (10) emittiert wird, teilweise mit der Elektronenwolke (I; II) überlappt, die von der mindestens einen zweiten Elektronenstrahlemissionsquelle (36) emittiert wird, wobei die Elektronenwolken (I, III) zusammen eine vereinigte Elektronenwolke bilden.

13. Verfahren nach Anspruch 12, wobei es den Schritt des mindestens vorübergehenden Anordnens der Öffnung des Verpackungsbehälters innerhalb der vereinigten Wolke aufweist.

## Revendications

1. Dispositif de stérilisation destiné à stériliser des récipients (12) d'emballage à l'aide de faisceaux d'électrons, ledit dispositif de stérilisation comportant
au moins un premier émetteur (10) de faisceaux d'électrons prévu pour la stérilisation d'au moins l'intérieur du récipient (12) d'emballage, et
au moins un deuxième émetteur (36) de faisceaux d'électrons prévu pour la stérilisation d'au moins l'extérieur de ledit récipient (12) d'emballage,
la stérilisation intérieure du récipient (12) d'emballage étant effectuée dans des conditions où le premier émetteur (10) de faisceaux d'électrons et le récipient (12) d'emballage sont prévus pour effectuer un mouvement relatif l'un par rapport à l'autre, de telle façon qu'au moins une partie du premier émetteur (10) de faisceaux d'électrons, comportant une fenêtre (20) de sortie d'électrons, soit logée dans le récipient (12) d'emballage à travers une ouverture (34) dans le récipient (12) d'emballage, et
la stérilisation extérieure du récipient (12) d'emballage étant effectuée dans des conditions où le récipient d'emballage est prévu pour passer devant une fenêtre de sortie d'électrons (40) du ou des deuxièmes émetteurs (36) de faisceaux d'électrons,
ledit dispositif de stérilisation comportant un support rotatif (64) par rapport auquel le ou les premiers émetteurs (10) de faisceaux d'électrons sont disposés de telle façon qu'ils soient mobiles conjointement avec le support (64), et celui-ci comportant un moyen d'acheminement de récipients d'emballage prévu pour acheminer le récipient (12) d'emballage de manière synchrone avec le mouvement de rotation du support (64), en maintenant un axe longitudinal du premier émetteur (10) de faisceaux d'électrons aligné avec un axe longitudinal du récipient (12) d'emballage,
ledit dispositif de stérilisation comportant deux deuxièmes émetteurs (36) de faisceaux d'électrons, disposés l'un en face de l'autre de telle manière que les récipients (12) d'emballage puissent passer entre eux, les deux deuxièmes émetteurs (36) de faisceaux d'électrons ayant leurs axes centraux longitudinaux parallèles entre eux, mais les deux deuxièmes émetteurs (36) de faisceaux d'électrons étant positionnés au moins partiellement sous le support (64) et ayant leurs axes centraux longitudinaux inclinés d'un angle par rapport aux axes longitudinaux du premiers émetteurs (10) de faisceaux d'électrons et à un arbre central du support (64).

2. Dispositif de stérilisation selon la revendication 1, le moyen d'acheminement de récipients d'emballage étant prévu pour déplacer le récipient (12) d'emballage par rapport au premier émetteur (10) de faisceaux d'électrons entre une première position dans laquelle le récipient (12) d'emballage et le premier émetteur (10) de faisceaux d'électrons ne sont pas en interaction entre eux et une deuxième position dans laquelle le récipient (12) d'emballage et le premier émetteur (10) de faisceaux d'électrons sont en interaction complète entre eux.

3. Dispositif de stérilisation selon la revendication 2, le support (64) comportant une position (68) d'introduction de récipients d'emballage et une position (70) d'extraction de récipients d'emballage, et ledit moyen d'acheminement de récipients d'emballage, une fois acheminé un récipient (12) d'emballage de la position (68) d'introduction de récipients d'emballage à la position (70) d'extraction de récipients d'emballage, étant en même temps prévu pour déplacer le récipient (12) d'emballage par rapport au premier émetteur (10) de faisceaux d'électrons de la première position à la deuxième position et le ramener la première position, pour stériliser ainsi l'intérieur du récipient (12) d'emballage.

4. Dispositif de stérilisation selon l'une quelconque des revendications précédentes, plusieurs premiers émetteurs (10) de faisceaux d'électrons étant répartis uniformément à la périphérie du support (64).

5. Dispositif de stérilisation selon l'une quelconque des revendications précédentes, celui-ci comportant deux deuxièmes émetteurs (36) de faisceaux d'électrons, disposés l'un en face de l'autre de telle manière que les récipients (12) d'emballage puissent passer entre eux, et leurs fenêtres de sortie d'électrons faisant face aux récipients d'emballage, les deux deuxièmes émetteurs (36) de faisceaux d'électrons ayant leurs axes centraux longitudinaux parallèles entre eux, et un des deux deuxièmes émetteurs de faisceaux d'électrons étant positionné au niveau ou au voisinage d'un arbre central (66) du support (64), et l'autre deuxième émetteur de faisceaux d'électrons étant disposé au voisinage de la périphérie du support (64).

6. Dispositif de stérilisation selon l'une quelconque des revendications 3 à 5 qui précèdent, les deux deuxièmes émetteurs (36) de faisceaux d'électrons étant disposés par rapport au support (64) de telle façon que la stérilisation extérieure soit effectuée avant que les récipients (12) d'emballage n'arrivent à la position (70) d'extraction.

7. Dispositif de stérilisation selon l'une quelconque des revendications précédentes, la stérilisation intérieure du récipient (12) d'emballage se terminant lorsque le récipient (12) d'emballage passe devant le ou les deuxièmes émetteurs (36) de faisceaux d'électrons de telle façon qu'un nuage (III) d'électrons du premier émetteur (10) de faisceaux d'électrons chevauche au moins partiellement un nuage (I ; II) d'électrons du ou des deuxièmes émetteurs (36) de faisceaux d'électrons, lesdits nuages d'électrons (I, III) formant ensemble un nuage combiné d'électrons, et l'ouverture (34) du récipient d'emballage étant au moins temporairement positionnée à l'intérieur du nuage combiné (I, III).

8. Dispositif de stérilisation selon l'une quelconque des revendications précédentes, ledit premier émetteur (10) de faisceaux d'électrons étant prévu pour stériliser au moins d'éventuelles surfaces de contact mutuel entre le récipient (12) d'emballage et ledit moyen d'acheminement de récipients d'emballage avant que ledit moyen d'acheminement de récipients d'emballage ne soit amené au contact du récipient (12) d'emballage ou lorsque les moyens d'acheminement d'emballage libèrent le récipient d'emballage.

9. Dispositif de stérilisation selon l'une quelconque des revendications précédentes, un ou plusieurs deuxièmes émetteurs (36) de faisceaux d'électrons étant disposés au voisinage du support (64), la fenêtre de sortie d'électrons (40) étant orientée vers le centre du support (64), et le moyen d'acheminement de récipients d'emballage étant prévu pour faire tourner chaque récipient (12) d'emballage autour de son propre axe au moins lorsqu'il passe devant ledit ou lesdits deuxièmes émetteurs (36) de faisceaux d'électrons.

10. Dispositif de stérilisation selon l'une quelconque des revendications précédentes, au moins deux deuxièmes émetteurs (36) de faisceaux d'électrons étant disposés, avec leurs fenêtres de sortie d'électrons (40) l'une en face de l'autre, de telle manière que les récipients (12) d'emballage puissent passer entre eux, et de telle manière que la stérilisation extérieure soit effectuée lorsque les récipients (12) d'emballage n'ont plus aucune interaction avec le premier émetteur (10) de faisceaux d'électrons.

11. Procédé de stérilisation de récipients d'emballage à l'aide de faisceaux d'électrons en utilisant un dispositif de stérilisation comportant un support rotatif (64) par rapport auquel le ou les premiers émetteurs (10) de faisceaux d'électrons sont disposés de telle façon qu'ils soient mobiles conjointement avec le support (64), et celui-ci comportant un moyen d'acheminement de récipients d'emballage prévu pour acheminer le récipient (12) d'emballage de manière synchrone avec le mouvement de rotation du support (64), en maintenant un axe longitudinal du premier émetteur (10) de faisceaux d'électrons aligné avec un axe longitudinal du récipient (12) d'emballage, ledit dispositif de stérilisation comportant deux deuxièmes émetteurs (36) de faisceaux d'électrons, disposés l'un en face de l'autre de telle manière que les récipients (12) d'emballage puissent passer entre eux, les deux deuxièmes émetteurs (36) de faisceaux d'électrons ayant leurs axes centraux longitudinaux parallèles entre eux, mais les deux deuxièmes émetteurs (36) de faisceaux d'électrons étant positionnés au moins partiellement sous le support (64) et ayant leurs axes centraux longitudinaux inclinés d'un angle par rapport aux axes longitudinaux du premiers émetteurs (10) de faisceaux d'électrons et un arbre central du support (64), le procédé comportant les étapes consistant à
stériliser au moins l'intérieur du récipient d'emballage à l'aide d'au moins un premier émetteur de faisceaux d'électrons et
stériliser au moins l'extérieur dudit récipient d'emballage à l'aide d'au moins un deuxième émetteur de faisceaux d'électrons, et
la stérilisation intérieure du récipient d'emballage étant effectuée dans des conditions où le premier émetteur de faisceaux d'électrons et le récipient d'emballage sont prévus pour effectuer un mouvement relatif l'un par rapport à l'autre, de telle façon qu'au moins une partie du premier émetteur de faisceaux d'électrons, comportant une fenêtre de sortie d'électrons, soit logée dans le récipient d'emballage à travers une ouverture dans le récipient d'emballage, et la stérilisation extérieure du récipient d'emballage étant effectuée dans des conditions où le récipient d'emballage est prévu pour passer devant une fenêtre de sortie d'électrons du ou des deuxièmes émetteurs de faisceaux d'électrons.

12. Procédé selon la revendication 11, le procédé comportant l'étape consistant à finaliser la stérilisation intérieure de telle façon qu'un nuage (III) d'électrons émis à partir du premier émetteur (10) de faisceaux d'électrons chevauche partiellement le nuage (I ; II) d'électrons émis à partir du ou des deuxièmes émetteurs (36) de faisceaux d'électrons, lesdits nuages d'électrons (I, III) formant ensemble un nuage combiné d'électrons.

13. Procédé selon la revendication 12, celui-ci comportant l'étape consistant à positionner au moins temporairement l'ouverture du récipient d'emballage à l'intérieur du nuage combiné.
